# EUROPEAN PATENT APPLICATION

(11) **EP 4 018 916 A1**
(43) Date of publication of application: **29.06.2022**
(21) Application number: 20862182.1
(22) Date of filing: 27.08.2020
(51) Int. Cl.: A61B 3/032

(54) **VISUAL FUNCTION TESTING DEVICE**

(30) Priority: 12.09.2019 JP 2019166297
(71) Applicant: Mitsui Chemicals, Inc., Tokyo 105-7122 (JP)
(72) Inventor: SUZUKI, Kenji, Sodegaura-shi, Chiba 299-0265 (JP)
(74) Representative: Epping - Hermann - Fischer
(86) International application number: PCT/JP2020/032331
(87) International publication number: WO 2021/049308

(57) **Abstract**

The present invention comprises a visual target display unit (100) which displays a visual target image (B) for causing a subject (P) to focus on a visual target region (Ba), and a light emitting unit (200) which is arranged at the front side of the visual target display unit (100) such that a first region (Ba) of the visual target display unit (100) is exposed toward the subject (P) and which emits interfering light (C) from a second region (Ca) positioned around the first region (Ba).

## Description

### Technical Field

The present disclosure relates to a visual function test apparatus.

### Background Art

Optical members such as lenses and filters have optical characteristics such as spectral transmittance, chromatic aberration, and refractive index. Regarding those of the optical members which are for correcting the visual function of a person (for example, eyeglasses), it is necessary to select one having optical characteristics suitable for the visual function of each individual.

In recent years, in order to test the visual function of a subject in consideration of various light environments, a visual function test assuming disability glare has attracted attention (for example, see Patent Literature (hereinafter, referred to as "PTL" 1). The disability glare is a phenomenon that reduces the visual recognition ability of a visual system during when a visual object is being viewed when there is a high luminance light source (glare light source) near the visual object.

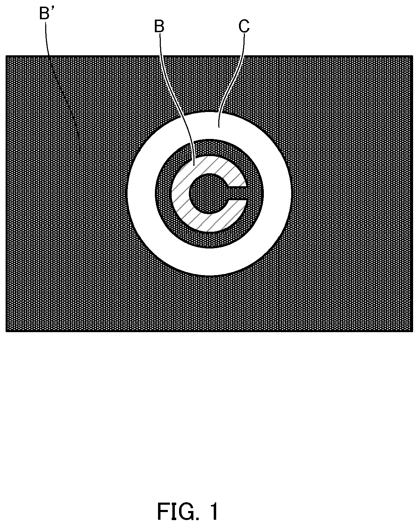

FIG. 1 illustrates an aspect of a visual target image displayed in a visual function test assuming disability glare.

This type of visual function test is conducted in a state where planar disturbing light C is generated at a position within the same field of view in proximity to visual target image B to which a subject is to pay attention. Note that, FIG. 1 illustrates an aspect in which an image of a Landolt ring is used as visual target image B, visual target image B is presented in a superimposed manner on background B' (e.g., black region) having a luminance different from the luminance of visual target image B, and a ring-shaped light emitting portion (hereinafter, also referred to as "glare portion") surrounding the periphery of visual target image B is used as the light source of disturbing light C.

In a state where disturbing light C as illustrated in FIG. 1 is present, the visual function test is conducted, for example, by sequentially displaying visual target images B having different luminances or colors to the subject while the luminance or color of background B' is kept constant. At this time, in order to test the visual function according to an aspect of disturbing light C, the luminance of disturbing light C or the stimulation value of color stimulation is also sequentially changed. In this aspect, the visual recognition ability is evaluated as a contrast discrimination threshold for discrimination of visual target image B with respect to background B'.

### Citation List

### Patent Literature

PTL 1
WO2018/012334

### Summary of Invention

### Technical Problem

Meanwhile, conventionally, as a visual function test apparatus for conducting this type of visual function test, a visual function test apparatus has been studied which displays, on the same single screen of a display (e.g., liquid crystal display), a visual target image and a glare portion that generates background light and disturbing light.

However, there is a problem that the visual function test apparatus according to the prior art is incapable of appropriately conducting the visual function test assuming the disability glare in real-life light environments such as viewing an outdoor visual object under sunlight, because the luminance of the disturbing light that can be generated is low and the viewing angle is limited.

Further, in such a visual function test apparatus, the spectral distributions of the visual target image and the disturbing light are limited to expressible spectral distributions of the display. It is thus difficult to make the spectral distributions different between the visual target image and the disturbing light, or to change the spectral distributions of either or both of them.

The present disclosure has been made in view of the above-mentioned problems, and an object thereof is to provide a visual function test apparatus capable of more appropriately conducting a visual function test assuming disability glare.

### Solution to Problem

Principally, the present disclosure that solves the aforementioned problems is
a visual function test apparatus that tests a visual function of a subject, the visual function test apparatus including:
a visual target displaying unit that displays, in a first region, a visual target image to which the subject is to pay attention; and
a light emitting unit that is disposed on a front surface side of the visual target displaying unit such that the first region of the visual target displaying unit is exposed to a subject side, the light emitting unit being configured to emit disturbing light from a second region located around the first region.

### Advantageous Effects of Invention

According to the visual function test apparatus of the present disclosure, it is possible to more appropriately conduct a visual function test assuming disability glare.

### Brief Description of Drawings

FIG. 1 illustrates an example of an aspect of a visual target image displayed in a visual function test assuming disability glare;
FIG. 2 illustrates an overall configuration of a visual function test apparatus according to Embodiment 1;
FIG. 3 illustrates an example of a visual target image displayed by the visual function test apparatus according to Embodiment 1;
FIGS. 4A to 4D illustrate examples of spectral distributions of disturbing light emitted from a light emitting unit according to Embodiment 1;
FIG. 5 illustrates an example of states of the visual target image with changed luminances in the visual function test apparatus according to Embodiment 1;
FIG. 6 illustrates an example of states of the visual target image and the disturbing light with changed colors in the visual function test apparatus according to Embodiment 1;
FIG. 7 illustrates an overall configuration of a visual function test apparatus according to Embodiment 2;
FIG. 8 illustrates a configuration of a visual target displaying plate according to Embodiment 2;
FIG. 9 illustrates an overall configuration of a visual function test apparatus according to Embodiment 3; and
FIG. 10 illustrates an example of the appearance of first disturbing light and second disturbing light seen when the subject visually recognizes the visual target image in the visual function test apparatus according to Embodiment 3.

### Description of Embodiments

Hereinafter, preferred embodiments of the present disclosure will be described in detail with reference to the appended drawings. In addition, in the present specification and drawings, components having substantially the same functions are provided with the same reference symbols, and redundant description will be omitted.

### (Embodiment 1)

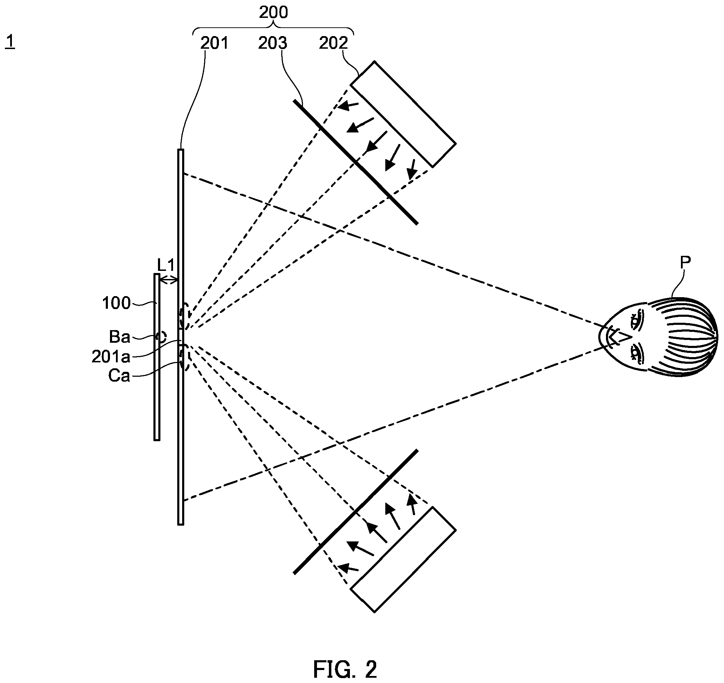

Hereinafter, an example of the configuration of a visual function test apparatus according to the present embodiment will be described with reference to FIGS. 2 to 4.

Visual function test apparatus 1 according to the present embodiment is typically used for conducting a visual function test assuming disability glare. The visual function test according to the present embodiment is conducted, for example, to obtain information that contributes to designing an optical member (e.g., an optical lens or an optical filter) that corrects the visual function of a subject.

### (Overall Configuration of Visual Function Test Apparatus)

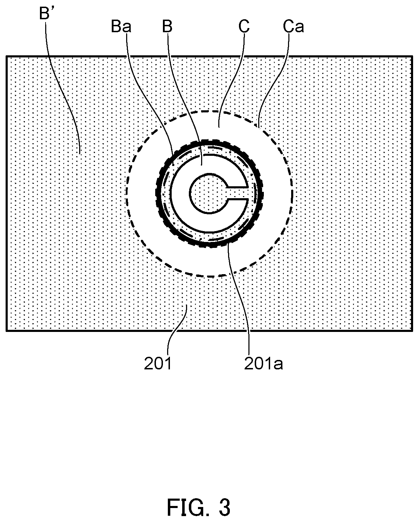

FIG. 2 illustrates an overall configuration of visual function test apparatus 1 according to the present embodiment. FIG. 3 illustrates an example of visual target image B displayed by visual function test apparatus 1 according to the present embodiment.

Visual function test apparatus 1 includes, for example, visual target displaying unit 100 and light emitting unit 200.

Visual target displaying unit 100 displays visual target image B to which subject P is to pay attention, in predetermined region (a circular region surrounded by a single-dot dashed line in FIG. 3, which is hereinafter referred to as a "visual target region" or a "first region") Ba. Visual target displaying unit 100 is installed, for example, at a distance of about 1 m from the position where subject P is seated, and at a height as high as the line of sight (for example, 1.2 m above the ground) of seated subject P.

Visual target displaying unit 100 is an electrically controlled display that electrically changes display contents, such as, for example, an organic EL display, an inorganic EL display, a liquid crystal display, a projector display, a laser display, or an electronic paper (here, visual target displaying unit 100 is a liquid crystal display).

Visual target displaying unit 100 typically displays, in background B' with a predetermined luminance and color, visual target image B of a different luminance and/or color being different from those of background B'. Visual target displaying unit 100 is configured to be capable of varying the luminance and/or spectral distribution of background B' and visual target image B.

Here, for example, the Landolt ring is used as visual target image B. In the visual recognition ability test, subject P judges the visual recognition ability in terms of discrimination based on, for example, whether or not the direction in which Landolt ring B lacks can be distinguished. Note that, when the direction of the cutout portion of the Landolt ring is to be answered to measure the ability to distinguish (= visual recognition ability), the visual recognition ability is referred to as the visual recognition ability in terms of "discrimination."

In addition, a sine wave fringe, a Gabor visual target image, a circular visual target image, an elliptical visual target image, a rectangular visual target image, an alphabetical visual target image, a Snellen visual target image, a hiragana visual target image, a numerical target image, an animal visual target image, Teller Acuity Cards (a visual target image for an infant which is formed according to a property of an infant preferring to view a fringe), or the like may be used as visual target image B.

Light emitting unit 200 generates planar disturbing light C at a position within the same field of view in proximity to visual target image B to which subject P is to pay attention. Light emitting unit 200 is disposed on the front surface side of visual target displaying unit 100 such that visual target region Ba of visual target displaying unit 100 is exposed on the subject P side. Also, light emitting unit 200 is positioned around visual target region Ba, and emits planar disturbing light C from region Ca surrounding visual target region Ba (a ring region surrounded by a broken line in FIG. 3, which is hereinafter referred to as a "glare portion" or a "second region"). In the visual function test, such disturbing light C causes subject P to have a state of disability glare.

Glare portion Ca is, for example, a ring-shaped white region surrounding the periphery of visual target image B. Glare portion Ca is set at a position in proximity to visual target image B, for example, at a predetermined distance away from visual target image B, with the background B' region being interposed in between. In addition, glare portion Ca may have a shape divided into a plurality of parts around visual target image B. In this case, glare portions Ca may be aligned into a shape such that the parts are arranged laterally symmetrically and vertically symmetrically with respect to visual target image B.

Light emitting unit 200 includes, for example, reflective plate 201 disposed on the front surface side of visual target displaying unit 100 and having opening 201a in a region corresponding to visual target region Ba, light source 202 that emits light from the front side (i.e., the subject P side) to glare portion Ca formed on reflective plate 201 to generate disturbing light C by the reflected light (hereinafter referred to as "disturbing light source 202"), and filter 203 that changes the spectral characteristics of the light emitted by disturbing light source 202 to change the spectral characteristics of disturbing light C.

Reflective plate 201 is formed of, for example, a black plate member, and is configured such that only glare portion Ca can reflect light coming from disturbing light source 202. Note that, reflection is a concept in a broad sense, including specular reflection and diffuse reflection.

In addition, reflective plate 201 is disposed in proximity to visual target displaying unit 100 such that binocular parallax occurring when subject P visually recognizes visual target image B through opening 201a is substantially eliminated. Distance L1 between reflective plate 201 and visual target displaying unit 100 is minimized by optimizing, for example, the size of opening 201a, the reference distance from visual target displaying unit 100 to subject P at the time of test, the distance between the eyes of subject P, and arrangement conditions under which the exposed portion of visual target displaying unit 100 is shaded by opening 201a against the light from the disturbing light source. Note that opening 201a is formed such that, for example, the opening edge of opening 201a is located at the position of the inner diameter of glare portion Ca (see FIG. 3).

Reflective plate 201 has, for example, a shape in which glare portion Ca is planar. Note that, the surface (including glare portion Ca) of reflective plate 201 may also be roughened. For example, recesses may be formed in a part of glare portion Ca of reflective plate 201 so as to converge, to subject P, the light emitted from disturbing light source 202. Thus, it is also possible to give a luminance distribution different depending on generation positions to disturbing light C generated from glare portion Ca, or to form a point-like glare light source of an extremely high luminance.

In addition, a transmissive member such as a glass plate may be fitted into the position of opening 201a of reflective plate 201. The transmissive member may be an optical filter having spectral characteristics, or may be an optical filter that reduces transmittance. Further, the transmissive member may also be a polished glass or a milky semi-transparent resin.

It is preferable that disturbing light source 202 be a light source including light of a wavelength in the visible range, and a white LED lamp is used here. Disturbing light source 202 is configured to be capable of generating disturbing light C having a luminance higher than the luminance of visual target image B with reference to the viewpoint of subject P.

Light emitting unit 200 is configured to be capable of varying the luminance and/or spectral distribution of disturbing light C. The luminance adjustment of disturbing light C is typically performed by increasing or decreasing the effective value of the luminous flux emitted from disturbing light source 202. Also, adjustment of the spectral distribution of disturbing light C is typically performed by changing the type of filter 203 disposed in the emission window of disturbing light source 202.

In the present embodiment, the luminance of visual target image B is made variable, for example, between 5 and 300 cd/m², the luminance of disturbing light C is made variable, for example, between 0 and 6,000 cd/m², and the luminance of background B' (the region between visual target image B and disturbing light C and the region outside disturbing light C) is 0 to 300 cd/m², for example, with reference to the viewpoint of subject P.

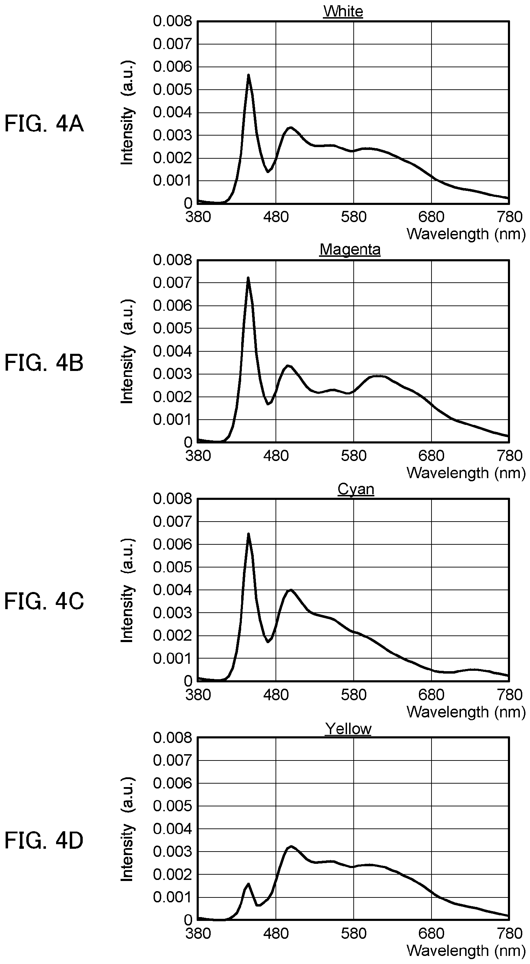

FIGS. 4A to 4D illustrate an example of the spectral distribution of disturbing light C emitted from light emitting unit 200.

FIG. 4A illustrates the spectral distribution of white disturbing light C, FIG. 4B illustrates the spectral distribution of magenta disturbing light C, FIG. 4C illustrates the spectral distribution of cyan disturbing light C, and FIG. 4D illustrates the spectral distribution of yellow disturbing light C. White disturbing light C in FIG. 4A has the spectral distribution of light emitted from disturbing light source 202 without using filter 203, and disturbing light C of the color in each of FIG. 4B, FIG. 4C, and FIG. 4D is obtained by converting the spectral distribution of light emitted from disturbing light source 202 via filter 203 of each color.

In the visual function test, for example, subject P visually views visual target image B displayed on visual target displaying unit 100, and the visual recognition ability of subject P to recognize visual target image B (e.g., whether the subject is able to see/is unable to see visual target image B, or feels/does not feel the glare, or the like) is tested. The light environment at this time is, for example, an indoor environment in which the outside light is blocked by a dark curtain, and the indoor illumination is turned on or off, and is a predetermined light environment in which the spectral distribution, intensity, or the like of light is managed by an inspector or the like.

The test for checking the disability glare is typically conducted after the test for checking the size of visual target image B which subject P can visually recognize in the absence of disturbing light C is conducted.

In the test for checking the disability glare, visual target images B having respective different luminances or colors are sequentially presented to subject P by visual target displaying unit 100 in a state in which disturbing light C is generated by light emitting unit 200, and subject P is asked to answer whether or not visual target image B can be visually recognized in each state. At this time, the luminances or colors of disturbing light C may also be sequentially changed in order to test the visual function depending on the modes of disturbing light C.

Note that, the visual function test is conducted by computer control (not illustrated) based on, for example, answers of subject P with respect to discrimination of Landolt rings, and visual target displaying unit 100 and light emitting unit 200 are controlled by the computer.

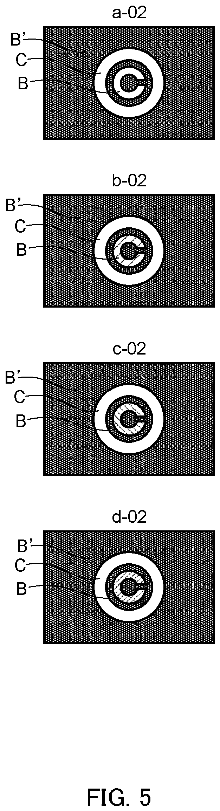

FIG. 5 illustrates an example of the states of visual target image B with changed luminances. FIG. 5 illustrates a mode in which the luminance of visual target image B is changed sequentially to a lower value in the order of a-02, b-02, c-02, d-02 in the state where background B' has a predetermined luminance and disturbing light C of a predetermined luminance is generated. Note that, visual target image B used at this time is, for example, of a visual recognition limit size for subject P checked in a preliminary test. Further, visual target image B used at this time is an image having a higher luminance than background B'.

In general, the visual recognition ability decreases due to the disability glare as a result of disturbing light C added. In this visual function test, for example, the luminance of visual target image B at the time when subject P answers "unable to visually recognize" is stored as a test result.

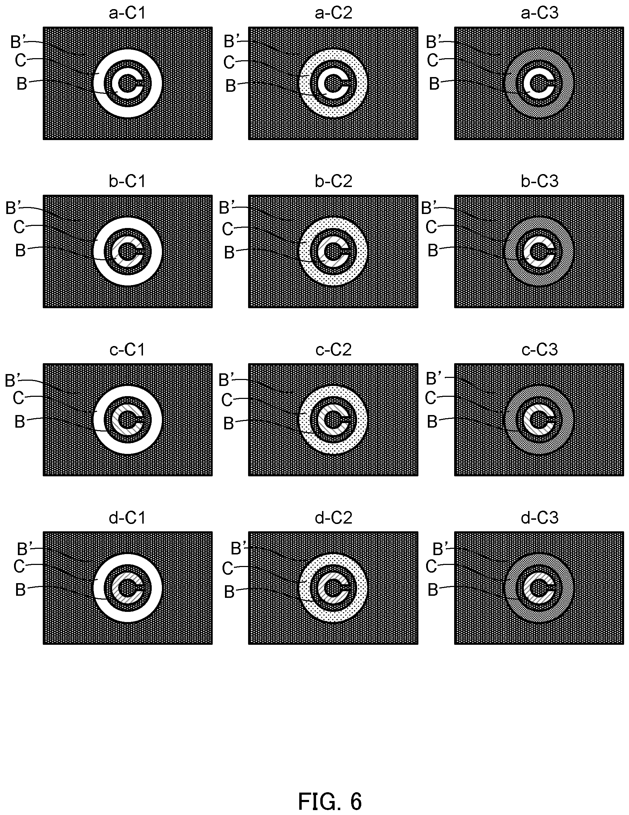

FIG. 6 illustrates an example of the states of visual target image B and disturbing light C with changed colors. FIG. 6 illustrates four modes in the case where the color of disturbing light C is set to colors illustrated in FIG. 4. The color of visual target image B is adjusted and controlled so as to be the same as disturbing light C.

Through such a test, for example, the visual recognition ability of subject P to recognize each color is checked. In this visual function test, for example, the luminance of visual target image B at the time when subject P answers "unable to visually recognize" is recorded as a test result for each color.

See, for example, PTL 1 for details of the visual function test.

As described above, visual function test apparatus 1 according to the present embodiment includes: visual target displaying unit 100 that displays, in visual target region Ba, visual target image B to which subject P is to pay attention; and light emitting unit 200 disposed on the front surface side of visual target displaying unit 100 such that first region Ba (the visual target region) of visual target displaying unit 100 is exposed on the subject P side, light emitting unit 200 being configured to emit planar disturbing light C from second region Ca surrounding the periphery of first region Ba, in which visual target displaying unit 100 displays visual target image B with a first luminance higher than the luminance of the background, and light emitting unit 200 emits disturbing light C with the a second luminance higher than the first luminance.

As is understood, according to visual function test apparatus 1 according to the present embodiment, it is possible to conduct the visual function test in a state where disturbing light C having a higher luminance than the luminance of visual target image B is generated. In addition, this makes it possible to independently adjust the luminance and/or spectral distribution of each of visual target image B and disturbing light C. It is thus possible to more appropriately conduct the visual function test assuming the disability glare.

Further, in visual function test apparatus 1 according to the present embodiment, since light emitting unit 200 (reflective plate 201) that generates disturbing light C is configured separately from visual target displaying unit 100 that generates visual target image B, various light environments can be reproduced. For example, by roughening a part of the surface of reflective plate 201, it is also possible to impart, to disturbing light C generated from glare portion Ca, a luminance distribution depending on the generation position.

Note that, as an example of visual function test apparatus 1, the above embodiment has been described in relation to the aspect in which visual target displaying unit 100 and light emitting unit 200 are capable of varying both the luminance and color. However, visual target displaying unit 100 and light emitting unit 200 may be configured to be capable of varying only the luminance or only the color.

### (Embodiment 2)

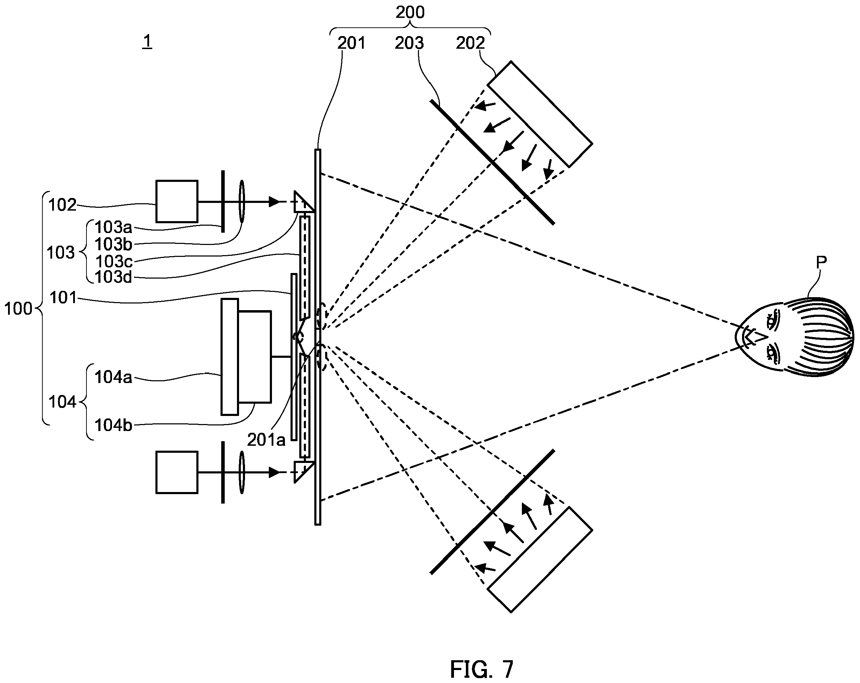

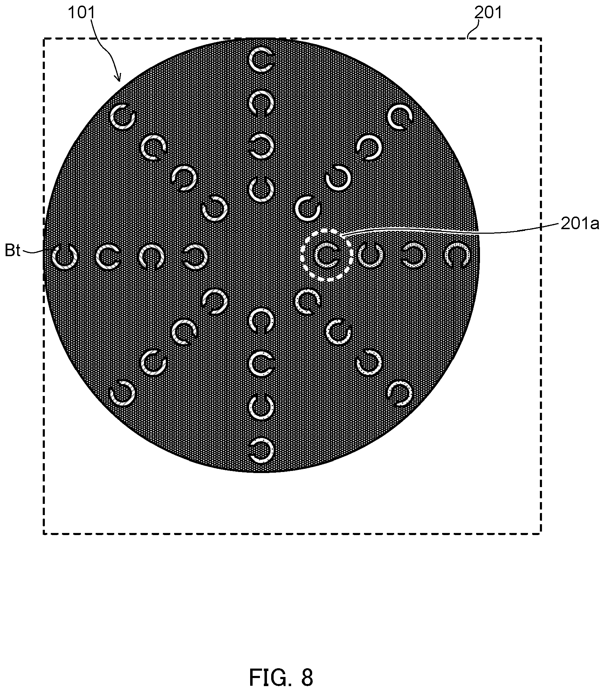

Next, a configuration of visual function test apparatus 1 according to Embodiment 2 will be described with reference to FIGS. 7 and 8.

Visual function test apparatus 1 according to the present embodiment differs from Embodiment 1 in the configuration of visual target displaying unit 100. The description of the configuration common to Embodiments 1 and 2 is omitted.

FIG. 7 illustrates an overall configuration of visual function test apparatus 1 according to the present embodiment. FIG. 8 illustrates a configuration of visual target displaying plate 101 according to the present embodiment.

Visual target displaying unit 100 according to the present embodiment includes visual target displaying plate 101 having a front surface on which patterns Bt corresponding to visual target images B are printed in advance, light source 102 (hereinafter, referred to as "visual target light source 102"), optical system 103 for guiding, to the front surface side of visual target displaying plate 101, light emitted from visual target light source 102, and drive section 104 for moving visual target displaying plate 101.

Visual target displaying plate 101 as seen in plan view is, for example, a circular plate-like member (see FIG. 8). A plurality of gray to white patterns Bt corresponding to visual target images B are formed on the front surface side of visual target displaying plate 101 in a gray background. Note that each of patterns Bt corresponding to visual target images B is applied and formed by, for example, a printing method.

FIG. 8 illustrates an aspect in which a plurality of patterns Bt are formed radially. Note that, a plurality of patterns Bt formed on visual target displaying plate 101 include, for example, patterns Bt with respective different reflectances to light emitted from visual target light source 102. Thus, visual target images B with different luminances depending on patterns Bt used among the plurality of patterns Bt can be achieved.

Visual target displaying plate 101 is covered with light emitting unit 200 such that any one of the plurality of patterns Bt is exposed to the outside through opening 201a. In addition, visual target displaying plate 101 reflects the light emitted from visual target light source 102, and allows subject P to visually recognize visual target image B through opening 201a.

Visual target light source 102 is, for example, a white LED lamp.

Optical system 103 includes filter 103a for converting the light emitted from visual target light source 102 into light having a desired spectral distribution, lens 103b for converting diffused light emitted from visual target light source 102 into parallel light, prism 103c for changing the traveling direction of the light sent out from lens 103b, and light guide plate 103d for guiding the light sent out from prism 103c to the front surface of visual target displaying plate 101. Note that, light guide plate 103d guides the light sent out from prism 103c to the position of visual target image B exposed to the outside through opening 201a.

The light emitted from visual target light source 102 passes through filter 103a, lens 103b, prism 103c, and light guide plate 103d, and is emitted to patterns Bt corresponding to visual target images B printed on visual target displaying plate 101. Accordingly, the light emitted from visual target light source 102 is reflected by visual target displaying plate 101, and subject P visually recognizes visual target image B by the reflected light.

Drive section 104 moves the position of visual target displaying plate 101 with respect to reflective plate 201. Specifically, drive section 104 includes linear motion stage 104a for moving visual target displaying plate 101 in the lateral direction with respect to light emitting unit 200 in a state in which the plate surface of visual target displaying plate 101 is parallel to the plate surface of reflective plate 201, and rotation stage 104b for rotating and moving visual target displaying plate 101 with respect to light emitting unit 200 in a state in which the plate surface of visual target displaying plate 101 is parallel to the plate surface of reflective plate 201. Thus, one of the plurality of patterns Bt of visual target images B formed on visual target displaying plate 101 is selectively exposed to the outside through opening 201a.

As described above, according to visual function test apparatus 1 according to the present embodiment, it is possible to display visual target image B of high luminance that is difficult to be achieved with an existing display (e.g., a liquid crystal display).

As an example of visual target displaying unit 100, the above embodiment has been described in relation to the aspect in which visual target image B is visually recognized by subject P using reflected light, but instead, an aspect may be employed in which visual target image B is visually recognized by subject P using transmitted light. In this case, visual target displaying unit 100 may be composed of visual target displaying plate 101 in which pattern Bt corresponding to visual target image B is formed in advance from a transmissive member, and light source 202 for emitting light from the rear surface side of visual target displaying plate 101.

Further, the above embodiment has been described in relation to the aspect of one example of drive section 104 in which the position of visual target displaying plate 101 is moved with respect to reflective plate 201. Instead of this, an aspect may also be employed in which visual target displaying plate 101 is moved with respect to the position of reflective plate 201.

### (Embodiment 3)

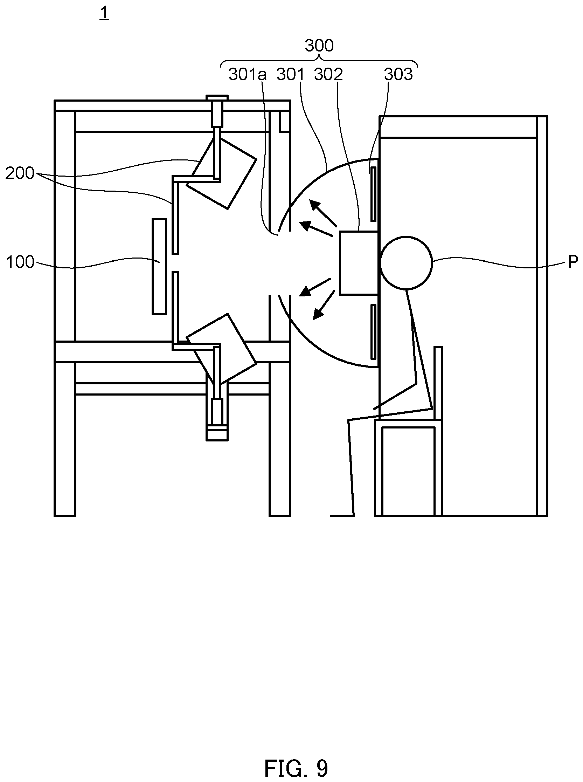

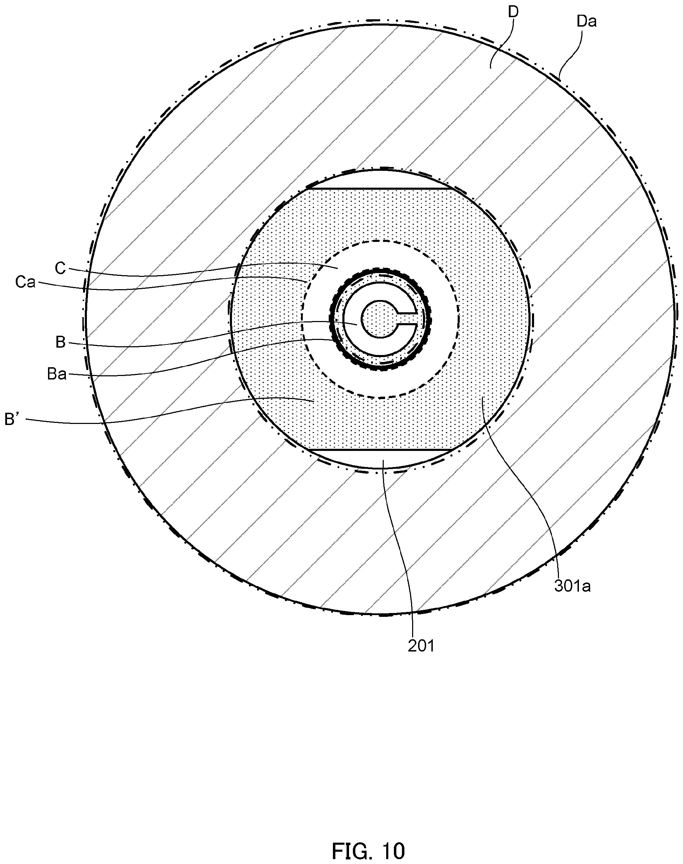

Next, a configuration of visual function test apparatus 1 according to Embodiment 3 will be described with reference to FIGS. 9 and 10.

Visual function test apparatus 1 according to the present embodiment differs from Embodiment 1 in that visual function test apparatus 1 according to the present embodiment further includes second light emitting unit 300. For convenience of description, light emitting unit 200 described in relation to Embodiment 1 is referred to as "first light emitting unit 200," and disturbing light C generated by first light emitting unit 200 is referred to as "first disturbing light C."

FIG. 9 is a side view illustrating the overall configuration of visual function test apparatus 1 according to the present embodiment. FIG. 10 illustrates an example of the appearance of first disturbing light C and second disturbing light D at the time when subject P visually recognizes visual target image B in visual function test apparatus 1 according to the present embodiment.

Second light emitting unit 300 is disposed in front of first light emitting unit 200 such that first region Ba in which visual target displaying unit 100 displays visual target image B and second region Ca in which light emitting unit 200 generates first disturbing light C are exposed to the outside, and second light emitting unit 300 emits second disturbing light D toward the subject P side from the outer peripheral region of second region Ca (the ring region surrounded by a double-dot dashed line denoted by "Da" in FIG. 10).

First disturbing light C generated by first light emitting unit 200 assumes a situation in which light of high luminance enters the central field of view of a person. However, in real life, there is a situation in which light enters not only the central field of view but also the peripheral field of view. Second light emitting unit 300 reproduces or simulates, as second disturbing light D, such light as that entering the peripheral field of view. Note that, examples of the light entering the peripheral field of view includes sunlight entering an eyeball when a person is present outdoors, reflected light from an object existing outdoors, and the like.

Second light emitting unit 300 includes second reflective plate 301, second light source 302, and reflective mirror 303.

Second reflective plate 301 has a dome shape (also referred to as a bowl shape) that bulges to the opposite side of subject P. Second reflective plate 301 is disposed in front of the eyes of subject P, and, when viewed from subject P, is in a state in which inner surface Da (meaning the inner surface of the dome shape; the same applies hereinafter) of second reflective plate 301 can be visually recognized. Note that, inner surface Da of second reflective plate 301 typically is white in order to be capable of reflecting light from second light source 302 at uniform spectral characteristics, and in addition, is formed from a highly reflective material in order to increase the reflectance.

Second reflective plate 301 has opening 301a at the top of the dome shape, and is configured such that first region Ba of visual target displaying unit 100 and second region Ca of light emitting unit 200 are exposed to the outside through opening 301a. That is, a state is achieved in which first region Ba of visual target displaying unit 100 and second region Ca of light emitting unit 200 are visually recognized from the reference position of subject P through opening 301a of second reflective plate 301, and inner surface Da of second reflective plate 301 is visually recognized around opening 301a from the reference position of subject P. Note that, second reflective plate 301 is typically disposed in front of the eyes of subject P.

Second light source 302 is disposed in front of second reflective plate 301, i.e., on the subject P side, and emits light to second reflective plate 301 from the front side to generate second disturbing light D from the inner surface of second reflective plate 301 by the reflected light. Second light source 302, for example, emits light to entire inner surface Da of second reflective plate 301 to generate planar second disturbing light D by reflection from entire inner surface Da of second reflective plate 301. Such second disturbing light D serves as disturbing light that enters the peripheral field of view of subject P who is visually recognizing first region Ba (i.e., visual target image B) in the central field of view.

Reflective mirror 303 is disposed in front of second reflective plate 301, (that is, on the subject P side) so as to face second reflective plate 301. Reflective mirror 303 reflects again, toward second reflective plate 301, a portion of the reflected light generated when second light source 302 emits light from the front side to the inner surface of second reflective plate 301. Accordingly, by utilizing multiple reflection between second reflective plate 301 and reflective mirror 303, it is possible to generate second disturbing light D traveling from the entire inner surface of second reflective plate 301 toward the subject P side with high luminance and uniformly regardless of the position of the inner surface of second reflective plate 301.

In the present embodiment, the luminance of visual target image B is made variable, for example, between 30 and 3,000 cd/m², the luminance of disturbing light C is made variable, for example, between 0 and 10,000 cd/m², and the luminance of background B' (the region between visual target image B and disturbing light C and the region outside disturbing light C) is 30 to 3,000 cd/m², for example, with reference to the viewpoint of subject P. In addition, second light emitting unit 300 can generate second disturbing light D whose luminance is variable, for example, between 0 and 10,000 cd/m² with reference to the viewpoint of subject P.

As described above, in visual function test apparatus 1 according to the present embodiment, it is possible to generate disturbing light D that enters the peripheral field of view of subject P. It is thus possible to conduct the visual function test in consideration of more diverse light environments.

Note that, the above embodiment has been described as an example of visual function test apparatus 1 in relation to the aspect in which first light emitting unit 200 and second light emitting unit 300 are separately implemented. However, the function of second light emitting unit 300 may be implemented by first light emitting unit 200. In this case, first reflective plate 201 may be configured to have a dome shape, and first reflective plate 201 may have a function of generating both first disturbing light C and second disturbing light D.

Further, with respect to the above embodiment, one example of second light emitting unit 300 has been described in relation to the configuration in which second disturbing light D is generated from entire inner surface Da of second reflective plate 301. However, second light emitting unit 300 may be configured to generate second disturbing light D from only a partial region of inner surface Da of second reflective plate 301.

While concrete examples of the present invention have been described in detail above, those examples are mere examples and do not limit the scope of the appended claims. The techniques disclosed in the scope of the appended claims include various modifications and variations of the concrete examples exemplified above.

The disclosure of Japanese Patent Application No. 2019-166297 dated September 12, 2019 including the specification, drawings and abstract is incorporated herein by reference in its entirety.

### Industrial Applicability

According to the visual function test apparatus of the present disclosure, it is possible to more appropriately conduct a visual function test assuming disability glare.

### Reference Signs List

1 Visual function test apparatus
100 Visual target displaying unit
101 Visual target displaying plate
102 Light source
103 Optical system
103a Filter
103b Lens
103c Prism
103d Light guide plate
104 Drive section
104a Linear motion stage
104b Rotation stage
106 Light guide plate
200 Light emitting unit
201 Reflective plate
201a Opening
202 Light source
203 Filter
300 Second light emitting unit
301 Second reflective plate
301a Opening (second opening)
302 Second light source
303 Reflective mirror
P Subject
B Visual target image
B' Background
C Disturbing light
Ba First region (Visual target region)
Ca Second region (glare portion)
Bt Pattern of visual target image
D Second disturbing light

## Claims

1. A visual function test apparatus that tests a visual function of a subject, the visual function test apparatus comprising:
a visual target displaying unit that displays, in a first region, a visual target image to which the subject is to pay attention; and
a light emitting unit that is disposed on a front surface side of the visual target displaying unit such that the first region of the visual target displaying unit is exposed to a subject side, the light emitting unit being configured to emit disturbing light from a second region located around the first region.

2. The visual function test apparatus according to claim 1, wherein:
the visual target displaying unit displays the visual target image with a first luminance, and
the light emitting unit emits the disturbing light having a second luminance higher than the first luminance.

3. The visual function test apparatus according to claim 1 or 2, wherein:
the light emitting unit has an opening formed to expose the first region of the visual target displaying unit to the subject side, and
the light emitting unit is disposed in proximity to the visual target displaying unit such that binocular parallax occurring when the subject visually recognizes the visual target image through the opening is substantially eliminated.

4. The visual function test apparatus according to any one of claims 1 to 3, wherein
the light emitting unit is configured to include a reflective plate and a light source, the reflective plate being disposed on the front surface side of the visual target displaying unit and having an opening at the first region, the light source being configured to emit light from a front side to the second region of the reflective plate to generate the disturbing light by reflected light resulting from the light emitted.

5. The visual function test apparatus according to claim 4, wherein
the light emitting unit includes a filter for changing a spectral characteristic of the light emitted by the light source.

6. The visual function test apparatus according to any one of claims 1 to 5, wherein
the light emitting unit is configured to be capable of varying luminance and/or spectral distribution of the disturbing light.

7. The visual function test apparatus according to any one of claims 1 to 6, wherein
the visual target displaying unit is configured to be capable of varying luminance and/or spectral distribution of the visual target image.

8. The visual function test apparatus according to any one of claims 1 to 7, wherein
the visual target displaying unit is an electrically controlled display for electrically changing display contents.

9. The visual function test apparatus according to any one of claims 1 to 7, wherein
the visual target displaying unit is configured to include a visual target displaying plate and a light source, the visual target displaying plate having a front surface on which a pattern corresponding to the visual target image is formed, the light source being configured to emit light from a front surface side of the visual target displaying plate.

10. The visual function test apparatus according to any one of claims 1 to 7, wherein the visual target displaying unit is configured to include:
a visual target displaying plate in which a pattern corresponding to the visual target image is formed from a transmissive member; and
a light source for emitting light from a rear surface side of the visual target displaying plate.

11. The visual function test apparatus according to claim 9 or 10, further comprising:
a drive section that moves the visual target displaying unit or the light emitting unit such that one of a plurality of the patterns corresponding to a plurality of the visual target images formed on or in the visual target displaying plate is exposed to the subject side through the opening.

12. The visual function test apparatus according to any one of claims 1 to 11, wherein
the visual function of the subject is tested by sequentially changing luminance and/or spectral distribution of the visual target image to be displayed on the visual target displaying unit.

13. The visual function test apparatus according to any one of claims 1 to 12, wherein
the visual function of the subject is tested by sequentially changing luminance and/or spectral distribution of the disturbing light caused to be emitted by the light emitting unit.

14. The visual function test apparatus according to any one of claims 1 to 13, further comprising:
a second light emitting unit disposed between the subject and the light emitting unit, the second light emitting unit being configured to emit second disturbing light that enters an eye of the subject from around the visual target image and the disturbing light when the subject visually recognizes the visual target image.

15. The visual function test apparatus according to claim 14, wherein:
the second light emitting unit includes a dome-shaped second reflective plate and a second light source, the dome-shaped second reflective plate bulging on a side opposite to the subject and having a second opening formed at a top portion on the side opposite to the subject, the second light source being configured to emit light to an inner surface of the dome-shaped second reflective plate to generate the second disturbing light by reflected light resulting from the light emitted, and
the first region of the visual target displaying unit and the second region of the light emitting unit are visually recognized from a reference position of the subject through the second opening, and the inner surface of the dome-shaped second reflective plate is visually recognized around the second opening from the reference position of the subject.

16. The visual function test apparatus according to claim 15, wherein
the second light emitting unit further includes a reflective mirror for reflecting again a portion of the reflected light to the dome-shaped second reflective plate, the reflected light being generated when the second light source emits light to the inner surface of the dome-shaped second reflective plate.
